# EUROPEAN PATENT APPLICATION

(11) **EP 0 631 785 A1**
(43) Date of publication of application: **04.01.1995**
(21) Application number: 94109311.4
(22) Date of filing: 16.06.1994
(51) Int. Cl.: A61K 35/02, A61K 9/08, A61K 9/14

(54) **Mineral ion solution, anti-human immunodeficiency virus agent, and aids-treating agent**

(30) Priority: 01.07.1993 JP 163598/93; 18.10.1993 JP 259802/93
(71) Applicant: HATUTIO CO., LTD, Tokyo (JP)
(72) Inventor: Nakayama, Genichiro, Iwafune-gun, Niigata-ken (JP); Yamamoto, Naoki, Shibuya-ku, Tokyo (JP)
(74) Representative: Strehl Schübel-Hopf Groening & Partner

(57) **Abstract**

Herein disclosed are a novel anti-HIV agent and a novel AIDS-treating drug, both comprising, as an active ingredient, a specific mineral ion solution, or a concentrate, a diluted solution or the solid matter thereof, said specific mineral ion solution having been obtained by keeping sandy tuff (starting mineral material) under warm and humid conditions to effect efflorescence of mud-yellow oxidized soil on the material, collecting the resultant oxidized soil and subsequently extracting it with water.

## Description

### BACKGROUND OF THE INVENTION

### (Field of the Invention)

This invention relates to an anti-human immunodeficiency virus agent (anti-HIV agent) and a drug for treating AIDS (acquired immunodeficiency syndrome), each containing a specific mineral ion solution or the solid matter thereof as an active ingredient, as well as to such a mineral ion solution or solid matter thereof per se and a process for producing such a mineral ion solution.

### (Prior Art)

It has been revealed that an AIDS-treating drug AZT (azidothymidine: a reverse transcriptase inhibitor) can retard onset and progress of AIDS significantly. However, AZT has some drawbacks such as development of chronic toxicity resulting from its prolonged administration and generation of drug-resistant AIDS virus mutants. On the other hand, a therapeutic means in which DDI (dideoxyinosine) and AZT are used in combination has recently been approved, but this means also causes serious side effects such as acute pancreatitis and the like. Each of these drugs has poor selectivity, because they are mononucleosides and exert influences upon not only reverse transcriptase but also human polymerases.

### (Problems to be Resolved by the Invention)

In view of such technical background, it is an object of the present invention to develop and provide a new anti-HIV agent, as well as an AIDS-treating agent, which is free from the aforementioned problems.

With the aim of achieving the above object, the inventors of the present invention have conducted intensive studies and found a distinct anti-HIV activity in a certain mineral ion solution or the solid matter thereof which has been obtained by allowing sandy tuff as the starting mineral material to undergo weathering and extracting, with water, the mud-yellow oxidized soil formed on the surface, and have accomplished the present invention on the basis of these findings.

### SUMMARY OF THE INVENTION

Accordingly, the present invention relates to an anti-human immunodeficiency virus agent and an AIDS-treating drug, each comprising as an active ingredient a certain mineral ion solution or the solid matter thereof obtained by allowing sandy tuff (starting mineral material) to undergo weathering and extracting, with water, the mud-yellow oxidized soil effloresced on the tuff surface.

It also relates to a process for producing such certain mineral ion solution, as well as to such a solution per se.

Incidentally, by the solid matter of a solution is meant the solute substances, because they form a solid residue when the solution is evaporated to dryness.

### DETAILED DESCRIPTION OF THE INVENTION

First of all, a process for the production of the mineral ion solution to be used as an active ingredient of the anti-HIV agent and AIDS-treating drug of the present invention will be described.

The mineral ion solution may be produced for example in the following manner.

That is, the mineral material to be used as the starting material is tuff, inter alia, sandy tuff produced for example in the area of Asahi-mura, Iwafune-gun, Niigata-ken (Niigata Prefecture), Japan.

The weathering treatment according to the present invention may be carried out for example in the following manner.

Firstly, such mineral material is subjected to preliminary weathering. This can be effected for example by allowing mined mineral material to stand packed in a hemp sack or as it is, in the field under such a condition that it is protected from rain and dew. The preliminary weathering is terminated when mud-yellow oxidized soil is slightly effloresced on the surface of the starting mineral material. Required time for the preliminary weathering may be about 6 months, though it varies depending on the weathering conditions.

Next, the thus slightly weathered material resultant from the preliminary weathering is kept in a warm and humid environment to effect additional formation of oxidized soil. The term warm and humid environment as used herein means such a warm and humid environment that mud-yellow oxidized soil is further effloresced on the surface of the preliminarily weathered mineral material, which can be realized, e.g., by keeping the material for example under the conditions that the indoor temperature, the material temperature and the material water content are controlled at 25 to 35°C, 20 to 25°C and 30 to 40%, respectively. When the mineral material is kept indoor under such warm and humid conditions for a period of about 6 months, mud-yellow oxidized soil is effloresced in a sufficient amount on the surface of the mineral material.

Next, the thus effloresced oxidized soil is collected. In this instance, the starting mineral material remaining after collection of the oxidized soil again produces sufficient amounts of oxidized soil when it is kept under the same warm and humid conditions for such a short period of time as about 15 days. Accordingly, this oxidized soil is also collected. In this manner, oxidized soil can be collected thereafter twice a month over about one year.

Finally, the thus collected oxidized soil is extracted with water with no particular difficulty. As a matter of course, it is preferable to use pure water (distilled water for example). In order to carry out such extraction thoroughly, the oxidized soil is added to hot water of for example 60°C, the mass is mixed and then allowed to stand for a while (2 hours for example), and the resultant liquid phase (mineral ion solution) and the solid phase (oxidized soil residue after extraction) are subsequently separated from each other by decantation. In this instance, the oxidized soil residue after extraction can be used as a raw material of cosmetic drugs, baths and the like.

The mineral ion solutions thus obtained have a reddish brown color for example and a pH value of 1 to 3. Such a mineral ion solution when tested gave the following analytical results as for the test items (mg/L); iron ^{... ...} 2,830; calcium ^{... ...} 251; sodium ^{... ...} 458; potassium ^{... ...} 1.1; magnesium ^{... ...} 601; arsenic (as As₂O₃) ^{... ...} 15; copper ^{... ...} 16.2; zinc ^{... ...} 101; manganese ^{... ...} 35.4; silicic acid (as SiO₂) ^{... ...} (not detected); aluminium ^{... ...} 841; fluorine ^{... ...} 32; boric acid ^{... ...} (not detected); free carbonic acid (CO₂) ^{... ...} 3,700; chlorine ion ^{... ...} 30; phosphate radical ^{... ...} 9.7; sulfate radical ^{... ...} 15,900; and a pH value of 2.2 and a specific gravity (15°C) of 1.019.

Since the thus obtained mineral ion solution is dense in concentration as it is and has a low pH value, it is used as a mother (or stock) ion liquid and applied to practical use after appropriately diluted as occasions demand. Also, it may be concentrated into a denser liquid for convenience sake of transportation or distribution, or drug compounding. And, in an extreme case, it may be evaporated to dryness under such mild conditions that the useful components are not denatured, and the resultant dry matter after pulverized is put to various uses. It may of course be diluted as occasions demand.

Next will be described the anti-HIV agent of the present invention which comprises the thus obtained mineral ion solution or the solid matter thereof as an active ingredient.

The anti-HIV activity of the mineral ion solution of the present invention will be evident later from Example 2.

The anti-HIV agent of the present invention having such an anti-HIV activity can be used for the disinfection of things which are contaminated or possibly contaminated with HIV, such as syringes, surgical tools such as surgical knives and the like, surgical gloves, surgical uniforms and surgical masks, as well as those which are stained with blood and the like body fluids of HIV carriers. In other words, it can be used as an HIV disinfectant.

Process per se for the preparation of the anti-HIV agent of the present invention as an HIV disinfectant is not particularly limited, and any known preparation process, including additives such as fillers and the like, may be employed, with the proviso that the aforementioned mineral ion solution including its concentrated and diluted products, or the solid matter thereof is used as an active ingredient.

With regard to an illustrative mode of the incorporation of the mineral ion solution as an active ingredient, an anti-HIV agent may be prepared using a mother ion liquid obtained in the aforementioned manner as it is or after diluting it as occasion demands. However, when convenience of transportation or distribution is taken into consideration, the anti-HIV agent of the present invention may also be prepared by employing, as an active ingredient, a mineral concentrate or a mineral ion dry powder as it is or after diluting it as occasions demand, the concentrate having been obtained by concentrating such a mother ion liquid into a denser liquid, and, in an extreme case, the dry powder having been obtained by concentrating such a mother ion liquid to dryness under such mild conditions that the useful components are not denatured. And, such a preparation process is also included in the illustrative mode of the incorporation of the mineral ion solution or the solid matter thereof of the present invention, as an active ingredient.

Disinfection of HIV contamination making use of the anti-HIV agent prepared in this way can be carried out with no particular difficulty, for example by dipping HIV-contaminated utensils in the anti-HIV agent of the present invention or by spraying the inventive anti-HIV agent to HIV-contaminated materials.

Finally, the AIDS-treating drug of the present invention which comprises the aforementioned mineral ion solution or the solid matter thereof as an active ingredient will be described, as follows.

As mentioned in the foregoing, the anti-HIV activity of the mineral ion solution of the present invention will again be evident later from Example 2.

One of the greatest advantages of the AIDS-treating drug of the present invention is that it can be administered orally, and the process per se for preparing dosage forms of the drug suitable for use in the oral administration is not particularly limited and can be effected by employing any known preparation process, including additives such as fillers and the like, with the proviso that the aforementioned mineral ion solution or the solid matter thereof is used as an active ingredient. For example, the inventive oral dosage forms may, like conventional oral dosage forms, be prepared by using, as an active ingredient, the aforementioned mineral ion solution (including its diluted or concentrated product) or dried powder of its solid matter as such or after diluting it as occasions demand and mixing the active ingredient with pharmaceutically acceptable carriers such as organic or inorganic solid or liquid fillers, the dried powder having been obtained by concentrating and drying a mineral ion solution under such mild conditions that the useful components are not denatured. These oral administration preparations can be in the dosage form of solids such as tablets, granules, powders, capsules and the like or of liquids such as solutions, suspensions, syrups, emulsions and the like. If necessary, these preparations may contain an auxiliary, a stabilizer, a moistening agent and other usually used additives. In addition, since the active ingredient of the therapeutic drug of the present invention is strongly acidic depending on its concentration, the drug should be prepared in such a manner that the pH value does not interfere with suitable oral administration and does not give too much stimulation to the inner walls of digestive tracts.

The AIDS-treating drug of the present invention can be applied to a patient after onset of the syndrome to inhibit the propagation of HIV and reconstruct the immunological system or to a carrier before onset of the syndrome to inhibit and retard the onset (in this case, if the onset of the carrier's syndrome could be inhibited throughout his or her life, it means that the carrier would make prolonged life and die a natural death).

The AIDS-treating drug of the present invention should be administered in such an amount (effective dose) that its effects can be obtained in the above usage, which in general may be approximately 0.1 mg to 20 g in terms of solid matter of the mineral ion solution of the present invention per a day per an adult of 60 kg in body weight, though the dose varies depending on the age and the like of each patient.

In this connection, the AIDS-treating drug of the present invention did not show acute toxicity when tested.

### Examples

The following examples are provided to further illustrate the present invention.

### Example 1 (Production of mineral ion solution)

Sandy tuff (produced in the area of Asahi-mura, Iwafune-gun, Niigata Prefecture, Japan) was, as the starting mineral material, subjected to preliminary weathering by allowing the material to stand packed in a hemp sack and under a vinyl sheet cover, in the field for 6 months. At the time of the termination of this treatment, mud-yellow oxidized soil was slightly effloresced on the surface of the starting material.

After the preliminary weathering, the starting material was taken out from the hemp sack and kept in a room in which the temperature was automatically controlled at 30°C, with occasional spraying of slightly hot water to control the water content of the material being treated at a level of 30 to 40%. During this period, the temperature of the material was found to be 20 to 25°C. After 6 months of this treatment, the mud-yellow oxidized soil effloresced on the surface of the thus treated material was collected. In this instance, additional mud-yellow oxidized soil effloresced on the surface of the material could be collected repeatedly thereafter twice a month over about one year by keeping the material soil after collection of the first crop of the oxidized soil under the same warm and humid conditions.

Next, 12 kg of the thus collected oxidized soil was mixed with 10 L of distilled water at 60°C with stirring, and the mixture was allowed to stand for 2 hours and then subjected to solid-liquid separation by decantation.

The resulting liquid phase was purified by means of silk filtration to obtain 2.7 L of purified mineral ion solution (mother ion liquid) of reddish brown in color. This mother ion liquid was reddish brown in color and showed a pH value of 1.9.

### Example 2 (Examination of anti-HIV activity)

### (a) Outline of the examination:

The examination was carried out in accordance with the method of Pauwels *et al*. (Pauwels *et al*., *J*. *Virol*. *Methods*, vol.20, pp.309 - 321 (1988)).

Together with the mother ion liquid obtained in Example 1 and diluted in advance to various concentrations, cells of an HIV-infected human T cell line MT-4 (*Gann monogr*., vol.28, pp.219 - 228 (1982)) are added to a 96 well microtiter plate (2.5 × 10⁴ cells/well, MOI (multiplicity of infection), inoculated viruses/cell: 0.01) and incubated. In order to examine the cytotoxicity of the mother ion liquid against MT-4 cells, HIV-uninfected cells are incubated in the same manner together with the mother ion liquid of various concentrations.

After 5 days of culture at 37°C in a CO₂ incubator, the viable cells are estimated by the MTT method.

Anti-HIV activity is expressed as a concentration which protects 50% of the cell damage caused by the HIV infection (EC₅₀), and cytotoxicity is expressed as a 50% cell damage concentration of a test material (mother ion liquid) (CC₅₀). Also, effective coefficient (selectivity index (SI)) is expressed as CC₅₀/EC₅₀.

### (b) Examination results:

(i) Results of the examination of the HIV-infected human MT-4 cell line are shown in Table 1. The tested concentration, 20.000%, of (1) in the table corresponds to the 5-fold dilution to which the mother ion liquid obtained in Example 1 was diluted.

**Table 1**

| Examination results of HIV-infected human cells | | | | | |
|---|---|---|---|---|---|
| Concentration tested (%) | Measured values (OD) | | | | Effectiveness (%) |
| | background | intermediate value | average value | standard deviation | |
| (1) 20.0000 | 0.102 | 0.087 | 0.089 | 0.008 | - 98.00 |
| (2) 10.0000 | 0.058 | 0.114 | 0.108 | 0.013 | - 77.71 |
| (3) 5.0000 | 0.093 | 0.411 | 0.429 | 0.030 | - 2.86 |
| (4) 2.5000 | 0.059 | 0.741 | 0.799 | 0.087 | 101.14 |
| (5) 1.2500 | 0.040 | 0.844 | 0.851 | 0.059 | 136.00 |
| (6) 0.6250 | 0.030 | 0.695 | 0.717 | 0.042 | 96.29 |
| (7) 0.3125 | 0.023 | 0.598 | 0.600 | 0.007 | 70.57 |
| (8) 0.1563 | 0.018 | 0.548 | 0.518 | 0.052 | 57.71 |
| (9) 0.0781 | 0.014 | 0.442 | 0.431 | 0.021 | 28.57 |
| (10) 0 | 0.014 | 0.342 | 0.331 | 0.021 | 0.00 |

(ii) Results of the examination of the HIV-uninfected human MT-4 cell line are shown in Table 2. The tested concentrations are the same as those shown in Table 1.

**Table 2**

| Examination results of HIV-uninfected human cells | | | | | | |
|---|---|---|---|---|---|---|
| Conc. tested (%) | Measured values (OD) | | | | Viability of cells (%) | Cytotoxicity (%) |
| | background | intermediate value | average value | standard deviation | | |
| (1) | 0.067 | 0.110 | 0.090 | 0.034 | 7.30 | 92.70 |
| (2) | 0.066 | 0.066 | 0.071 | 0.014 | 1.02 | 98.98 |
| (3) | 0.102 | 0.159 | 0.188 | 0.044 | 9.34 | 90.66 |
| (4) | 0.062 | 0.489 | 0.483 | 0.044 | 63.36 | 36.64 |
| (5) | 0.043 | 0.594 | 0.603 | 0.013 | 81.46 | 18.54 |
| (6) | 0.036 | 0.590 | 0.594 | 0.021 | 81.90 | 18.10 |
| (7) | 0.028 | 0.651 | 0.646 | 0.008 | 91.97 | 8.03 |
| (8) | 0.022 | 0.648 | 0.657 | 0.013 | 92.41 | 7.59 |
| (9) | 0.021 | 0.645 | 0.659 | 0.022 | 92.12 | 7.88 |
| (10) | 0.019 | 0.697 | 0.691 | 0.008 | 100.00 | 0.00 |

(iii) From the above data, various kinds of concentrations and coefficient are calculated as shown in Table 3.

**Table 3**

| Summary of the examination results | |
|---|---|
| 50% effective concentration (EC₅₀) | 0.1301% |
| 90% effective concentration (EC₉₀) | 0.5275% |
| 50% cytotoxic concentration (CC₅₀) | 2.9675% |
| effective coefficient (SI = CC₅₀/EC₅₀) | 23 |

### (Effects of the Invention)

Novel anti-HIV agent and AIDS-treating drug are provided by the present invention.

## Claims

1. A process for producing a mineral ion solution which comprises allowing sandy tuff (starting mineral material) to undergo weathering and extracting, with water, the mud-yellow oxidized soil effloresced on the tuff surface.

2. The mineral ion solution obtainable by the process of Claim 1, and a concentrate, a diluted solution and the solid matter thereof.

3. An anti-human immunodeficiency virus agent which comprises, as an active ingredient, the mineral ion solution, the concentrate, the diluted solution or the solid matter, according to Claim 2.

4. The anti-human immunodeficiency virus agent of Claim 3 compounded by employing, as an active ingredient, the mineral ion solution, the concentrate, the diluted solution or the solid matter, according to Claim 2.

5. An AIDS-treating drug which comprises, as an active ingredient, the mineral ion solution, the concentrate, the diluted solution or the solid matter, according to Claim 2.

6. The AIDS-treating drug of Claim 5 compounded by employing, as an active ingredient, the mineral ion solution, the concentrate, the diluted solution or the solid matter, according to Claim 2.
